# EUROPEAN PATENT SPECIFICATION

(11) **EP 2 923 205 B1**
(45) Date of publication and mention of the grant of the patent: **15.05.2019**
(21) Application number: 13795229.7
(22) Date of filing: 22.11.2013
(51) Int. Cl.: G01N 33/564, G01N 33/68

(54) **ANALYSIS FOR ADULT-ONSET STILL'S DISEASE**
ANALYSE DES EINSETZENS VOM ADULTEN MORBUS STILL
ANALYSE DE LA MALADIE DE STILL SURVENANT CHEZ L'ADULTE

(30) Priority: 23.11.2012 EP 12194134
(43) Date of publication of application: 30.09.2015
(73) Proprietor: Baerlecken, Niklas, 50933 Köln (DE)
(72) Inventor: BAERLECKEN, Niklas, 30171 Hannover (DE); WITTE, Torsten, 30559 Hannover (DE)
(74) Representative: Taruttis, Stefan Georg
(86) International application number: PCT/EP2013/074453
(87) International publication number: WO 2014/079966

(56) References cited:
- WO-A2-2008/137835
- US-A1- 2007 184 444
- US-A1- 2011 207 613
- JOUEN F. ET AL.: "Diagnostic and prognostic values of anti glucose-6-phosphate isomerase antibodies in community-recruited patients with very early arthritis", CLIN. EXP. IMMUNOL., vol. 137, no. 3, September 2004 (2004-09), pages 606-611, XP55093984,
- BAERLECKEN N.T. ET AL.: "Adulter Morbus Still, Fieber, Diagnose und Therapie", Z. RHEUMATOL., vol. 71, no. 3, 15 April 2012 (2012-04-15) , pages 174-180, XP035045077, cited in the application
- BAERLECKEN N.T. ET AL.: "Association of ferritin autoantibodies with giant cell arteritis/polymyalgia rheumaticae", ANN. RHEUM. DIS., vol. 71, no. 6, June 2012 (2012-06), pages 943-947, XP009175245,
- BAERLECKEN N.T. ET AL.: "Antibodies against Drp-4 and macropain subunit C2 as a potential marker of AOSD", ARTHRIT. RHEUM., vol. 65, no. Suppl. 10, 1 October 2013 (2013-10-01), page s333, XP009175231,

### SEQUENCE LISTING

<110> Medizinische Hochschule Hannover
<120> Analysis for adult-onset Still's disease
<130> M1052PCT-EP
<140> EP13795229.7
   <141> 2013-11-22
<150> EP12194134
   <151> 2012-11-23
<160> 26
<170> BiSSAP 1.3
<210> 1
<400> 1
   000
<210> 2
   <211> 22
   <212> PRT
   <213> Artificial Sequence
<220>
   <223> synthetic peptide
<400> 2
   Arg Asn Gln Tyr Asp Asn Asp Val Thr Val Trp Ser Pro Gln Gly Arg
   1               5                   10                  15
   Ile His Gln Ile Glu Tyr
               20
<210> 3
<400> 3
   000
<210> 4
<400> 4
   000
<210> 5
<400> 5
   000
<210> 6
<400> 6
   000
<210> 7
<400> 7
   000
<210> 8
<400> 8
   000
<210> 9
<400> 9
   000
<210> 10
<400> 10
   000
<210> 11
<400> 11
   000
<210> 12
<400> 12
   000
<210> 13
<400> 13
   000
<210> 14
<400> 14
   000
<210> 15
<400> 15
   000
<210> 16
<400> 16
   000
<210> 17
<400> 17
   000
<210> 18
<400> 18
   000
<210> 19
<400> 19
   000
<210> 20
<400> 20
   000
<210> 21
<400> 21
   000
<210> 22
<400> 22
   000
<210> 23
<400> 23
   000
<210> 24
   <211> 263
   <212> PRT
   <213> Homo sapiens
<220>
   <223> macropain subunit C2
<400> 24
   Met Phe Arg Asn Gln Tyr Asp Asn Asp Val Thr Val Trp Ser Pro Gln
   1               5                   10                  15
   Gly Arg Ile His Gln Ile Glu Tyr Ala Met Glu Ala Val Lys Gln Gly
               20                  25                  30
   Ser Ala Thr Val Gly Leu Lys Ser Lys Thr His Ala Val Leu Val Ala
           35                  40                  45
   Leu Lys Arg Ala Gln Ser Glu Leu Ala Ala His Gln Lys Lys Ile Leu
       50                  55                  60
   His Val Asp Asn His Ile Gly Ile Ser Ile Ala Gly Leu Thr Ala Asp
   65                  70                  75                  80
   Ala Arg Leu Leu Cys Asn Phe Met Arg Gln Glu Cys Leu Asp Ser Arg
                   85                  90                  95
   Phe Val Phe Asp Arg Pro Leu Pro Val Ser Arg Leu Val Ser Leu Ile
               100                 105                 110
   Gly Ser Lys Thr Gln Ile Pro Thr Gln Arg Tyr Gly Arg Arg Pro Tyr
           115                 120                 125
   Gly Val Gly Leu Leu Ile Ala Gly Tyr Asp Asp Met Gly Pro His Ile
       130                 135                 140
   Phe Gln Thr Cys Pro Ser Ala Asn Tyr Phe Asp Cys Arg Ala Met Ser
   145                 150                 155                 160
   Ile Gly Ala Arg Ser Gln Ser Ala Arg Thr Tyr Leu Glu Arg His Met
                   165                 170                 175
   Ser Glu Phe Met Glu Cys Asn Leu Asn Glu Leu Val Lys His Gly Leu
               180                 185                 190
   Arg Ala Leu Arg Glu Thr Leu Pro Ala Glu Gln Asp Leu Thr Thr Lys
           195                 200                 205
   Asn Val Ser Ile Gly Ile Val Gly Lys Asp Leu Glu Phe Thr Ile Tyr
       210                 215                 220
   Asp Asp Asp Asp Val Ser Pro Phe Leu Glu Gly Leu Glu Glu Arg Pro
   225                 230                 235                 240
   Gln Arg Lys Ala Gln Pro Ala Gln Pro Ala Asp Glu Pro Ala Glu Lys
                   245                 250                 255
   Ala Asp Glu Pro Met Glu His
               260
<210> 25
   <211> 23
   <212> PRT
   <213> Artificial Sequence
<220>
   <223> synthetic peptide
<400> 25
   Phe Arg Asn Gln Tyr Asp Asn Asp Val Thr Val Trp Ser Pro Gln Gly
   1               5                   10                  15
   Arg Ile His Gln Ile Glu Tyr
               20
<210> 26
   <211> 22
   <212> PRT
   <213> Artificial Sequence
<220>
   <223> synthetic peptide section of DRP-4
<400> 26
   Pro Gly Gly Val Asp Val His Thr Arg Leu Gln Met Pro Val Leu Gly
   1               5                   10                  15
   Met Thr Pro Ala Asp Asp
               20

## Description

The present invention relates to an analytical process for use in diagnosing an analyte which is specific for adult-onset Still's disease, and to peptides for use in the analytical process. It is an advantage of the peptides and of the analytical process using the peptides that antibodies can be used as analytes specific for adult-onset Still's disease (AOSD), which antibodies can be detected in the serum of a suspect patient due to their specificity for the peptides, adding to or avoiding the hitherto necessary deduction of the diagnosis from a combination of several clinical symptoms.

### State of the art

Baerlecken and Schmidt, Zeitschrift für Rheumatologie, 71(3), pages 174-180 (2012), describe that AOSD is diagnosed by a multitude of clinical and diagnostic criteria, especially by recurring fever episodes and a number of indicators for autoimmune diseases as well as by high serum ferritin levels.

Jung et al. in Journal of Rheumatology, 37(5), pages 1029 - 1034 (2010) describe serum calprotectin as a marker for AOSD.

Jouen et al, Clin Exp Immunol 137, pages 606 - 611 (2004) describe that no significant association was found between the presence of antiglucose-6-phosphate isomerase antibodies and very early arthritis.

WO 2008/0137835 describes a large number of auto-antigens for prognosis of a large number of autoimmune disorders.

US 2011/0207613 A1 describes antibodies as biomarkers for lupus (SLE) against the auto-antigen ZMAT2, and further additional auto-antigens.

Baerlecken and Schmidt, Zeitschrift für Rheumatologie, 71, pages 174 - 180 (2012) give an overview over diagnostics for AOSD.

Baerlecken et al, Ann Rheum Dis, 71, pages 943 - 947 (2012) describe the association of ferritin auto-antibodies with giant cell arteritis and polymyalgia rheumatica.

US 2007/0184444 A1 describes 6464 protein sequences that may serve for the treatment of an immune related disorder.

Komiya et al. in Scand J Rheumatol 156 - 164 (2012) describe the upregulation of neutrophil CD64 in AOSD.

US 2003/0054421 A1 contains an extensive list of proteins, including proteasome subunit C3, all of which proteins are indicated to play a role in a large number of diseases.

### Object of the invention

It is an object of the invention to provide an alternative method for analysis of AOSD, as well as to provide a specific reactant to identify an analyte of AOSD.

### General description of the invention

The invention achieves the object by the features as defined in the claims, especially by an analytical process using specific peptides serving to detect antibodies in the serum of a patient, the antibodies serving as the analyte which is indicative of AOSD. These antibodies are therefore also referred to as auto-antibodies. Further, the invention provides the peptides for use as specific reactants to the antibodies, which are the analytes that are indicative of AOSD. The peptides used as reagents which are specific for antibodies indicative of AOSD in the analytical process have the advantage of allowing a differentiation of AOSD from other medical conditions involving fever, e.g. from inflammatory rheumatoid diseases or from infectious diseases or malignant diseases accompanied by fever.

The at least one peptide used in the analytical method for detecting antibodies in the serum of a patient, which at least one peptide binds antibody indicative of AOSD comprises or consists of SEQ ID NO: 2 . Preferably, the peptide is immobilized on a carrier, e.g. on the surface of a microtiter plate or a flow through channel, optionally linked by a linker to the carrier.

Optionally, the at least one peptide can be part of a larger protein, wherein preferably the at least one peptide forms the only epitope or antigenic section of the protein. In general, it is preferred that the least one peptide forms the only antigenic amino acid sequence to be contacted with the serum of the patient. Accordingly, it is preferred that the antigenic amino acid sequence of a protein comprising the least one peptide, or of the peptide comprises or consists at least of sections of SEQ ID NO: 2, e.g. SEQ ID NO: 24 (corresponding to the amino acid sequence of macropain subunit C2).

It is a special advantage of the peptide of SEQ ID NO: 2 that bound antibodies were detected in sera of all of those patients analysed which have been diagnosed with AOSD previously, but only to a lower concentration or not in sera from those patients having different inflammatory rheumatoid diseases or in patients having infectious diseases or malignant diseases accompanied by fever.

Preferably, the analytical method in addition to detecting the presence of antibody binding to SEQ ID NO: 2, the process comprises the step of detecting the presence of antibody in the serum, which antibody is specific for dihydropyrimidinase-related protein 4 (DRP-4), e.g. by detecting the presence of antibody binding a peptide of SEQ ID NO: 26.

Using a peptide of SEQ ID NO: 26 in an ELISA, antibody against DRP-4 was found in serum obtained from the majority of those patients previously identified as suffering from AOSD, but not in the remaining set of patients suffering from inflammatory rheumatoid diseases, and in none of the sera from patients suffering from infectious diseases or malignant diseases accompanied by fever.

Accordingly, it is preferred that the sample originates from a patient without an inflammatory rheumatoid disease and/or without an infectious disease or malignant disease accompanied by fever.

The process for analysis comprises or consists of the steps of
- providing a sample, preferably serum, e.g. cell-free blood serum, e.g. obtained by separating cells from serum from a blood sample of a patient,
- providing at least one peptide from the group comprising or consisting of SEQ ID NO:2, which peptide is optionally comprised in a protein, wherein preferably the antigenic amino acid section of the protein consists of the at least one peptide,
- contacting the sample, preferably serum, with the at least one peptide and
- detecting the presence of antibody which is bound to the at least one peptide, e.g. by removal of unbound antibody, e.g. by washing of the carrier to which the at least peptide is immobilized, followed by detecting bound antibody, e.g. by detecting the presence of antibody coupled to the carrier.
- Optionally, the sample additionally is contacted with DRP-4, preferably with a peptide of SEQ ID NO: 26, preferably immobilized, and the presence of antibody bound to DRP-4 is detected. Preferably, the sample is concurrently contacted with at least one peptide comprising or consisting of SEQ ID NO: 2 and with DRP-4, followed by sequential or concurrrent detection of the presence of antibody bound to the at least one peptide and of antibody bound to DRP-4.
- Preferably, the process comprises the step of comparing the concentration of antibody specific for a peptide of SEQ ID NO: 2 in the sample with the cut-off value of the concentration of antibody specific for the peptide of SEQ ID NO: 2 as determined in ROC analysis.
- Preferably, the further process comprises the step of comparing the concentration of antibody specific for DRP-4, e.g. of SEQ ID NO: 26, in the sample with the cut-off value of the concentration of antibody specific for DRP-4 as determined in samples from healthy persons, e.g. blood donors.

The cut-off value can generally be determined by the receiver operating characteristic curve (ROC) analysis in regard of the maximal sum of sensitivity plus specificity. Patients with AOSD were defined as the fraction of true positives out of the positives (True positive rate, TPR) and febrile disease patients were defined as the fraction of the false positives out of the negatives (False positive rate, FPR). Each ELISA was performed with 8 negative blood donors.

Presence of antibody bound to the peptide, optionally in addition the presence of antibody bound to DRP-4, indicates that the sample contains antibody which is a specific analyte indicative of AOSD, allowing to diagnose AOSD in the patient. Accordingly, the analytical process is preferably followed by treatment of the patient for AOSD when antibody bound to the peptide is determined.

The bound antibody can be detected by known methods, e.g. by using a secondary antibody specific for the heavy chain of the antibody, e.g. anti-human antibody, preferably coupled to a detectable label, e.g. an optically detectable label, followed by measuring the amount of label bound to the carrier after removal of unbound secondary antibody.

It has been found that the specificity of the analytical process for providing an indication for AOSD is enhanced when the patient has been diagnosed additionally with at least one of the parameters of arthritis, preferably detected by an imaging technique like ultrasound or magnet resonance imaging (MRI), fever, e.g. at least 39 °C, leukocytosis, e.g. at least 10.000/µl, neutrophilia, e.g. at least >80%, an elevated serum level of liver enzymes, e.g. aspartate aminotransferase and alanine aminotransferase, and combinations thereof. Accordingly, the sample preferably originates from a patient who has been diagnosed with having or diagnosed with being free from at least one of the parameters selected from the group of arthritis, fever, leukocytosis, neutrophilia, an eleveated serum level of ferritin, an elevated serum level of transaminases, e.g. aspartate aminotransferase and/or alanine aminotransferase, and combinations thereof.

### Detailed description of the invention

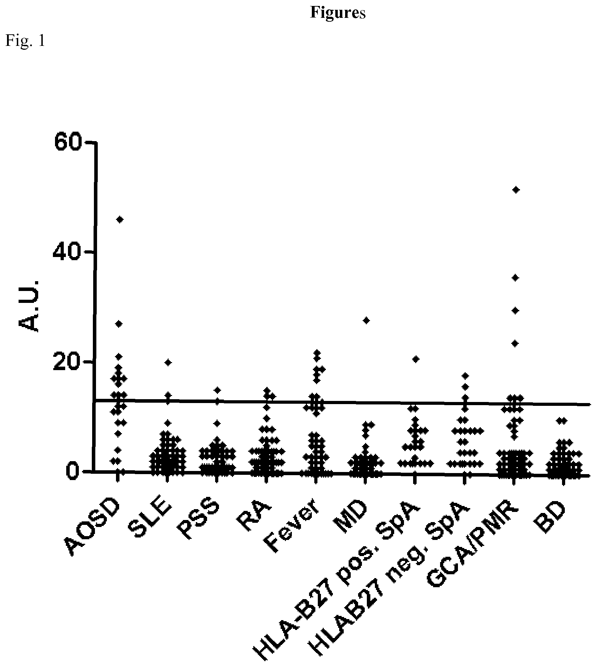

The invention is now described by way of examples with reference to the figures, wherein
- Figure 1 shows analytical results of ELISA assays using a peptide comprising a section of the macropain subunit C against sera from a variety of patients and
- Figure 2 shows analytical results of ELISA assays using a peptide comprising a section of the DRP-4 against sera from the variety of patients used in the analysis shown in Figure 1,
- Figure 3 shows analytical results of ELISA assays using a peptide comprising a section of the macropain subunit C or DRP-4 against sera from confirmed AOSD patients and healthy blood donors,
- Figure 4 shows the proportion of serum samples having antibody concentrations against macropain subunit C2 and against DRP-4 above the respective cut-off concentrations, for samples originating from patients with different diseases,
- Figure 5 shows serum antibody concentrations against macropain subunit C2 (MP) or against DRP-4 (DRP4) determined for patients diagnosed for AOSD, for patients with arthritis and patients without arthritis, and
- Figure 6 shows concentrations of antibody detected in serum of AOSD patients prior to and after 6 months following diagnosis of AOSD.

### Example 1: Detection of antibodies that are indicative of AOSD

In this example, 485 sera from patients and 156 negative control sera were analysed. Of these 388 patients, 72 were diagnosed according to Yamaguchi et al. as suffering from AOSD, and different inflammatory rheumatoid diseases or infectious diseases accompanied by recurring fever or by malignant diseases accompanied by recurring fever. In detail, sera from the following groups of patients were analysed: 56 patients suffered from systemic lupus erythematosus (SLE), 50 systemic juvenile arthritis (SJIA), 50 patients from primary Sjoegren's syndrome (PSS), 91 patients from rheumatoid arthritis (RA), 49 patients from infectious diseases and fever symptoms (fever), 52 patients from malignant diseases (MD), 55 patients with spondyloarthritis (SpA), polyangiitis with granulomatosis (GPA), 66 patients with giant cell arteritis and polymyalgia rheumatica (GCA/PMR, PMR/GCA). Sera from 156 disease-free blood donors (BD) were used as negative controls.

For the analysis, peptides comprising or consisting of SEQ ID NO: 2, optionally additionally peptide DRP-4, are separately immobilized on a carrier, alternatively as spaced apart spots on a flat substrate, or in separate wells of a microtiter plate. In detail, a peptide of SEQ ID NO: 25 was used, comprising SEQ ID NO: 2 plus an N-terminal addiditional phenylalanine. For analysis of the presence of antibody specific for DRP-4, a peptide of SEQ ID NO: 26 was used, corresponding to amino acids no. 67 to 88 of DRP-4 (DRP-4 A67-88).

For the analyis, an ELISA was performed in 96-well polystyrene microtiter plates available from Nunc (Denmark) under the name Maxisorb as carrier. 0.1µg peptide in TRIS-buffered saline (TBS) was added per well and incubated overnight for immobilisation. Unspecific binding sites of the carrier were saturated by unspecific blocking using 300µL TBS containing 1% bovine serum albumine (BSA) per well for 10 min. Contacting of serum to the immobilized peptides was performed according to standard practice, including serial dilution and control peptides. In detail, sera were diluted 1 : 100 in phosphate buffered saline (PBS) containing 5% BSA and added to the wells at 100µL after removal of blocking solution and incubated for 30min at room temperature. Following the incubation, the serum dilutions were removed and the wells were washed three times using 300mL TBS containing 0.02% Tween20 (TBS-T).

After the removal of unbound serum components by washing with buffer, bound antibody was detected by using labelled anti-human antibody and measuring the label. In detail, 100µL goat-anti-human antibody (IgG) labelled with horse raddish peroxidase (HRP), available from Jackson ImmunoResearch Europe, was added as a detection antibody at a dilution of 5 :100000, or at a dilution of 0.5µL in 10mL PBS supplemented with 10mL 5% BSA solution. Following an incubation for 30min at room temperature, unbound detection antibody was removed by washing the plates three times with PBS-T. The label was detected by the colour development using Tetramethylbenzidine (TMB, available from Thermo Fisher Scientific, Denmark) as a substrate for HRP for up to 30min according to the manufacturer's instructions, followed by spectrophotometry using an ELISA reader at 450nm.

In each ELISA, preferably in each microtiter plate, sera from 8 disease-free blood donors were included and used as a negative control. When assessing the detection results of the ELISA readings, 1 standard deviation measured for the blood donors was used as 1 arbitrary unit (A.U.). The detection result obtained for one of the patients diagnosed with AOSD was used as a cut-off (CO) value.

The results are given in the following table :

| | AOSD | FEVER | MD | GCA/PMR | SLE | PSS | RA | SpA | BD |
|---|---|---|---|---|---|---|---|---|---|
| MP | 52% | 22% | 1% | 12% | 5% | 4% | 3% | 9% | 0% |
| DRP-4 | 72% | 8% | 1% | 18% | 11% | 2% | 4% | 15% | 1% |
| MP + DRP-4 | 52% | 8% | 1% | 12% | 5% | 2% | 3% | 9% | 0% |

The results show that predominantly those 25 sera of patients that were previously diagnosed with AOSD bound to peptide of SEQ ID NO:25, showing the specificity of these peptides for detecting an antibody as an analyte indicative of AOSD in serum. In detail, 52% of these sera bound to the peptide of SEQ ID NO: 25. Further, a predominant proportion of the sera of patients that were previously diagnosed with AOSD bound to SEQ ID NO: 26, representing DRP-4. The data show that the analysis for AOSD is more specific when including the detection of antibody specific for DRP-4, e.g. detecting antibody specific for SEQ ID NO: 26, in addition to the detection of antibody specific for macropain subunit C2, especially for SEQ ID NO: 2.

As a general advantage, no antibody was bound to any of the peptides of SEQ ID NO: 2, nor to DRP-4, from sera of patients with infectious or malignant disease, each accompanied by recurring fever.

### Example 2 : Concentration of antibody in sera against a peptide of SEQ ID NO: 2 or against a peptide of SEQ ID NO: 26

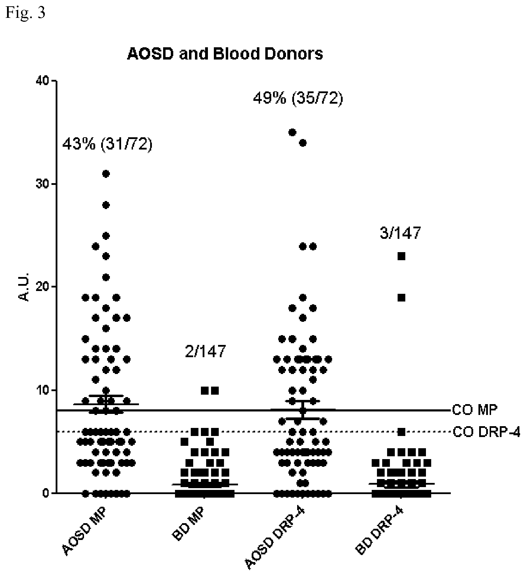

Concentrations of antibodies against a peptide of SEQ ID NO: 2 in sera from AOSD patients or from healthy blood donors were analysed according to Example 1 using a peptide of SEQ ID NO: 25. Figure 3 compares concentrations of antibody against the peptide of SEQ ID NO:25 (MP) in blood sera obtained from AOSD patients (AOSD), confirmed according to the Yamaguchi criteria and in blood sera from blood donors (BD), it was found that in 145 of 147 samples from blood donors (BD MP), the concentration of antibodies against SEQ ID NO:25 was below a cut-off value, whereas the concentration of such antibodies in 31 of 72 sera from AOSD patients was above the cut-off value (cut-off value for anti-macropain subunit-antibody, CO MP). Accordingly, it is generally preferred that the analysis comprises the step of quantifying the concentration of antibody specific for a peptide of SEQ ID NO: 2, and more preferably comparing this concentration to the cut-off value of the concentration determined in sera from healthy persons. A concentration of antibody specific for a peptide of SEQ ID NO: 2 above the cut-off value is indicative of AOSD. The cut-off value for SEQ ID NO: 25 in this embodiment of the analytical process by receiver operating characteristic (ROC) curve analysis in regard of the maximal sum of sensitivity plus specificity. Patients with AOSD were defined as the fraction of true positives out of the positives (True positive rate, TPR) and febrile disease patients were defined as the fraction of the false positives out of the negatives (False positive rate, FPR). Each ELISA was performed with 8 negative blood donors.

Further, Fig. 3 shows concentrations of antibody against DRP-4 (DRP-4), determined according to the method of Example 1, in the same sera of AOSD patients (AOSD DRP-4) and of healthy blood donors (BD DRP-4). It is found that in 35 of 72 sera of AOSD patients, the concentration of antibody specific for DRP-4 is above the cut-off value (cut-off value for anti-DRP-4-antibody, CO DRP-4) of this specific antibody determined in sera of healthy persons. Accordingly, it is generally preferred that the analysis comprises the step of quantifying the concentration of antibody specific for a peptide of SEQ ID NO: 26, and more preferably comparing this concentration to the cut-off value of the concentration determined in sera from healthy persons. A concentration of antibody specific for a peptide of SEQ ID NO: 26 above the cut-off value is indicative of AOSD. The cut-off value for DRP-4 in this embodiment of the analytical process is by receiver operating characteristic (ROC) curve analysis in regard of the maximal sum of sensitivity plus specificity. Patients with AOSD were defined as the fraction of true positives out of the positives (True positive rate, TPR) and febrile disease patients were defined as the fraction of the false positives out of the negatives (False positive rate, FPR). Each ELISA was performed with 8 negative blood donors.

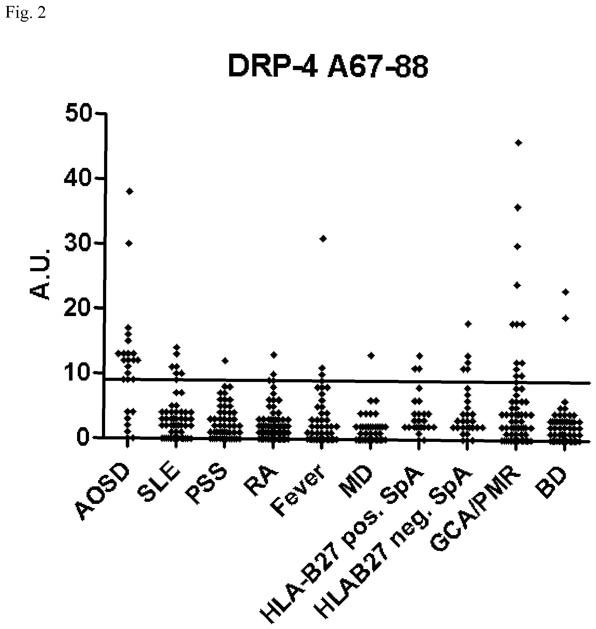

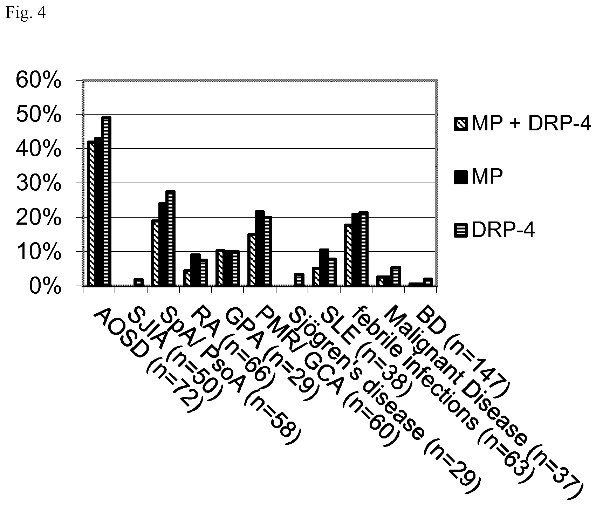

Fig. 4 shows the proportion (%) of sera with antibody concentrations against macropain subunit C2 and against DRP-4 above the respective cut-off concentrations for sera originating from patients with different diseases. The proportion of sera containing antibody concentrations above the cut-off value are shown for antibody against macropain subunit-antibody (MP central col.), against DRP-4 (DRP-4, right col.), and against both macropain subunit-antibody and DRP-4 (MP+DRP-4, left col.). These samples include further patients in addiditon to those of Fig. 2. The diseases in addition to those of Fig. 2 are systemic juvenilearthritis (SJIA), spondyloarthritis and psoriatic arthritis (SpA/PsoA), polyangiitis with granulomatosis (GPA), polymyalgia rheumatica and giant cell arteritis (PMR/GCA), or febrile infections. Antibody concentrations were determined as described in Example 1.

The data show that, e.g. for SpA/PsoA, 19% of samples had elevated (above cut-off value determined by ROC analysis as in Example 2) concentrations for both MP and DRP-4, and for 15% of samples of PMR/GCA, 18% of samples of febrile infections and 3% of malignant disease had elevated concentrations for both MP and DRP-4. For samples from AOSD patients, approx. 42% of samples had elevated concentrations for both MP and DRP-4. This shows that excluding samples from patients diagnosed with SpA/PsoA, PMR/GCA and febrile infections increases the specificity of the analytical process for AOSD. Accordingly, the sample of the process preferably in general excludes a sample originating from a patient diagnosed with SpA and/or PsoA, PMR and/or GCA or a febrile infection.

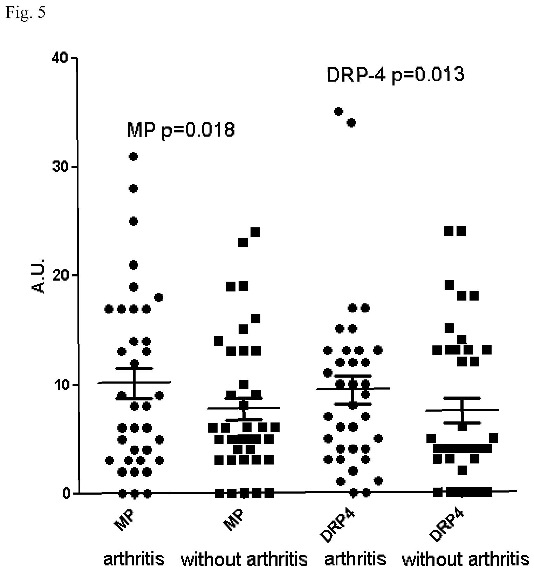

Fig. 5 shows concentrations of serum antibodies against a peptide of SEQ ID NO: 25 (MP) or against DRP-4 (DRP4) determined for patients diagnosed for AOSD according to the Yamaguchi criteria, for patients with arthritis and patients without arthritis. The Y-axis shows arbitrary units (A.U.) of antibody concentrations for relative comparison. The horizontal bar indicates the cut-off value determined by ROC analysis. The antibody concentrations bound to the peptide of SEQ ID NO: 25 (MP) or bound to DRP-4 (DRP4) show that in samples originating from patients who were diagnosed with arthritis, generally higher concentrations of auto-antibodies against these peptides are present, including a higher cut-off value.

Accordingly, these data show that in samples originating from patients diagnosed with arthritis, higher concentrations of auto-antibodies against SEQ ID NO: 25 (MP) and against DRP-4 (DRP4).

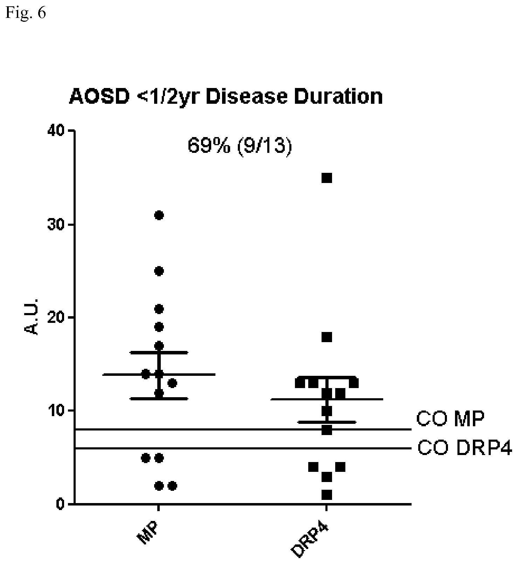

Fig. 6 shows antibody concentrations against macropain subunit C2 and against DRP-4 determined in serum samples originating from AOSD patients diagnosed for AOSD according to the Yamaguchi criteria for less than 6 months. Analyses for anti-macropain subunit C2-antibody (MP) and anti-DRP-4 antibody were performed according to Example 1. The results show that the anti-macropain subunit C2-antibody is a specific analyte for AOSD, and also that anti-DRP-4 antibody is a specific analyte for AOSD.

## Claims

1. Process for the analysis of an analyte specific for adult-onset Still's disease (AOSD) in a blood serum sample, comprising the steps of
- providing at least one peptide comprising SEQ ID NO : 2 ,
- contacting the sample with the peptide and
- detecting antibody bound to the peptide as the analyte.

2. Process according to claim 1, **characterized by** the peptide consisting of SEQ ID NO: 2.

3. Process according to one of the preceding claims, **characterized by** the sample being serum obtained from a patient who has been diagnosed not to have fever nor arthritis.

4. Process according to one of the preceding claims, **characterized in that** the at least one peptide is immobilized on a carrier and detection of antibody bound to the peptide is by contacting the carrier with a labelled anti-human antibody and measuring the label.

5. Process according to one of the preceding claims, **characterized in that** the peptide is bound to a linker.

6. Process according to one of the preceding claims, **characterized in that** the sample in addition is analysed for at least one of leukocytosis, neutrophilia, a high concentration of ferritin, and/or a high concentration of transaminases.

7. Process according to one of the preceding claims, **characterized in that** the sample excludes a sample originating from a patient diagnosed with spondyloarthritis (SpA) and/or psoriatic arthritis (PsoA), polymyalgia rheumatica (PMR) and/or giant cell arteritis (GCA) or a febrile infection.

8. Process according to one of the preceding claims, **characterized by** additionally detecting antibody specific for dihydropyrimidinase-related protein 4 (DRP-4).

9. Process according to claim 8, **characterized in that** antibody specific for DRP-4 is detected by contacting the sample with a peptide comprising or consisting of SEQ ID NO: 26 and detecting antibody bound to the peptide comprising or consisting of SEQ ID NO: 26.

10. Process according to one of the preceding claims, **characterized in that** the sample is additionally contacted with a peptide comprising or consisting of SEQ ID NO: 25.

11. Use of a peptide as a specific binding agent for an analyte which is specific for AOSD in a process for analysis of an analyte specific for AOSD, **characterized in that** the peptide comprises or consists of SEQ ID NO: 2.

12. Use according to claim 11, **characterized in that** a peptide comprising or consisting of SEQ ID NO: 26 is comprised as an additional peptide.

13. Use according to one of claims 11 to 12, **characterized in that** a peptide comprising or consisting of SEQ ID NO: 25 is comprised as an additional peptide for use as a specific binding agent which is specific for AOSD.

14. Use according to one of claims 11 to 13, **characterized in that** the peptide is immobilized on a carrier.

15. Use of a kit of parts in the analysis of serum from a patient for AOSD, comprising a peptide of SEQ ID NO: 26 and at least one peptide comprising or consisting of SEQ ID NO: 2 for use as specific binding agents for antibody from the serum as analytes which are specific for AOSD.

## Patentansprüche

1. Verfahren zur Analyse eines für adulten Morbus Still (AOSD) spezifischen Analyten in
einer Probe Blutserum, das die Schritte umfasst von
Bereitstellen wenigstens eines Peptids, das die SEQ ID NO: 2 umfasst,
Kontaktieren der Probe mit dem Peptid und
Nachweisen von Antikörper, der an das Peptid als dem Analyten gebunden hat.

2. Verfahren nach Anspruch 1, **dadurch gekennzeichnet, dass** das Peptid aus SEQ ID NO: 2 besteht.

3. Verfahren nach einem der voranstehenden Ansprüche, **dadurch gekennzeichnet, dass** die Probe Serum ist, die von einem Patienten erhalten wurde, für den diagnostiziert wurde, dass er weder Fieber noch Arthritis hat.

4. Verfahren nach einem der voranstehenden Ansprüche, **dadurch gekennzeichnet, dass** das wenigstens eine Peptid auf einem Träger immobilisiert ist und der Nachweis von an das Peptid gebundenem Antikörper durch Kontaktieren des Trägers mit einem markierten anti-humanen Antikörper und Messen der Markierung erfolgt.

5. Verfahren nach einem der voranstehenden Ansprüche, **dadurch gekennzeichnet, dass** das Peptid an einen Linker gebunden ist.

6. Verfahren nach einem der voranstehenden Ansprüche, **dadurch gekennzeichnet, dass** die Probe zusätzlich auf wenigstens eines von Leukozytose, Neutrozytose, eine hohe Konzentration an Ferritin und/oder eine hohe Konzentration von Transaminasen analysiert wird.

7. Verfahren nach einem der voranstehenden Ansprüche, **dadurch gekennzeichnet, dass** die Probe eine Probe ausschließt, die von einem Patienten stammt, für den Spondylarthritis (SpA) und/oder Arthritis psoriatica (PsoA), rheumatische Polymyalgie (PMR) und/oder Riesenzellartheriitis (GCA) oder eine fiebrige Infektion diagnostiziert wurde.

8. Verfahren nach einem der voranstehenden Ansprüche, **gekennzeichnet durch** zusätzliches Nachweisen von Antikörper, der für Dihydropyrimidinase-bezogenes Protein 4 (DRP-4) spezifisch ist.

9. Verfahren nach Anspruch 8, **dadurch gekennzeichnet, dass** der für DRP-4 spezifische Antikörper durch Kontaktieren der Probe mit einem Peptid nachgewiesen wird, das SEQ ID NO: 26 umfasst oder daraus besteht und Nachweisen des an das Peptid, das SEQ ID NO: 26 umfasst oder daraus besteht, gebundenen Antikörpers.

10. Verfahren nach einem der voranstehenden Ansprüche, **dadurch gekennzeichnet, dass** die Probe zusätzlich mit einem Peptid in Kontakt gebracht wird, das SEQ ID NO: 25 umfasst oder daraus besteht.

11. Verwendung eines Peptids als ein spezifisches Bindemittel für einen Analyten, der für AOSD spezifisch ist, in einem Verfahren zur Analyse eines für AOSD spezifischen Analyten, **dadurch gekennzeichnet, dass** das Peptid SEQ ID NO: 26 umfasst oder daraus besteht.

12. Verwendung nach Anspruch 11, **dadurch gekennzeichnet, dass** ein Peptid, das SEQ ID NO: 26 umfasst oder daraus besteht, als ein zusätzliches Peptid enthalten ist.

13. Verwendung nach einem der Ansprüche 11 bis 12, **dadurch gekennzeichnet, dass** ein Peptid, das SEQ ID NO: 25 umfasst oder daraus besteht, als ein zusätzliches Peptid umfasst ist, zur Verwendung als ein spezifisches Bindemittel, das für AOSD spezifisch ist.

14. Verwendung nach einem der Ansprüche 11 bis 13, **dadurch gekennzeichnet, dass** das Peptid auf einem Träger immobilisiert ist.

15. Verwendung eines Satzes von Teilen in der Analyse von Serum von einem Patienten für AOSD, das ein Peptid von SEQ ID NO: 26 und wenigstens ein Peptid umfasst, das SEQ ID NO: 2 umfasst oder daraus besteht, zur Verwendung als spezifische Bindemittel für Antikörper aus dem Serum als Analyten, die für AOSD spezifisch sind.

## Revendications

1. Processus d'analyse d'un analyte spécifique de la maladie de Still débutant à l'âge adulte (AOSD) dans un échantillon de sérum sanguin comprenant les étapes suivantes fournir au moins un peptide comprenant la SEQ ID NO: 2,
mettre en contact l'échantillon avec le peptide et
détecter un anticorps lié au peptide sous forme d'analyte.

2. Procédé selon la revendication 1, **caractérisé par** le peptide consistant en SEQ ID NO: 2.

3. Procédé selon l'une des revendications précédentes, **caractérisé en ce que** l'échantillon est du sérum obtenu chez un patient dont on a diagnostiqué l'absence de fièvre et d'arthrite.

4. Procédé selon l'une des revendications précédentes, **caractérisé en ce que** ledit au moins un peptide est immobilisé sur un support et **en ce que** la détection de l'anticorps lié au peptide se fait par mise en contact du porteur avec un anticorps antihumain marqué et mesuré du marqueur.

5. Procédé selon l'une des revendications précédentes, **caractérisé en ce que** le peptide est lié à un agent de liaison.

6. Procédé selon l'une des revendications précédentes, **caractérisé en ce que** l'échantillon est en outre analysé pour au moins une leucocytose, une neutrophilie, une concentration élevée de ferritine et/ou une concentration élevée de transaminases.

7. Procédé selon l'une des revendications précédentes, **caractérisé en ce que** l'échantillon exclut un échantillon provenant d'un patient chez lequel on a diagnostiqué une spondylar-thropathie (SpA) et/ou un psoriasis (PsoA), une polymyalgie rhumatismale (RPM) et/ou une artérite à cellules géantes (GCA) ou une infection fébrile.

8. Procédé selon l'une des revendications précédentes, **caractérisé en outre par** la détection d'un anticorps spécifique de la protéine 4 liée à la dihydropyrimidinase (DRP-4).

9. Procédé selon la revendication 8, **caractérisé en ce que** l'anticorps spécifique de DRP-4 est détecté par mise en contact de l'échantillon avec un peptide comprenant ou consistant en SEQ ID NO: 26, et détection d'un anticorps lié au peptide comprenant ou consistant en SEQ ID NO: 26.

10. Procédé selon l'une des revendications précédentes, **caractérisé en ce que** l'échantillon est en outre mis en contact avec un peptide comprenant ou consistant en SEQ ID NO: 25.

11. Utilisation d'un peptide en tant qu'agent de liaison spécifique pour un analyte spécifique de l'AOSD dans un procédé d'analyse d'un analyte spécifique de l'AOSD, **caractérisé en ce que** le peptide comprend ou consiste en SEQ ID NO: 2.

12. Procédé selon la revendication 11, **caractérisé en ce qu'**un peptide comprenant ou consistant en SEQ ID NO: 26 est compris en tant que peptide supplémentaire.

13. Utilisation selon l'une des revendications 11 ou 12, **caractérisé en ce qu'**un peptide comprenant ou consistant en SEQ ID NO: 25 est compris en tant que peptide supplémentaire destiné à une utilisation comme agent de liaison spécifique, lequel agent est spécifique à l'AOSD.

14. Utilisation selon l'une des revendications 11 à 13, **caractérisée en ce que** le peptide est immobilisé sur un porteur.

15. Utilisation d'un kit de pièces dans l'analyse du sérum d'un patient souffrant d'AOSD, comprenant un peptide de SEQ ID NO: 26 et au moins un peptide comprenant ou consistant en SEQ ID NO 2 destiné à une utilisation comme agent de liaison spécifique pour les anticorps provenant du sérum en tant qu'analytes spécifiques pour l'AOSD.
